# EUROPEAN PATENT APPLICATION

(11) **EP 0 641 563 A1**
(43) Date of publication of application: **08.03.1995**
(21) Application number: 93910346.1
(22) Date of filing: 17.05.1993
(51) Int. Cl.: A61K 31/335, A61K 9/08, A61K 9/00, C07D 303/48

(54) **REMEDY FOR CATARACT AND PRODUCTION THEREOF**

(30) Priority: 20.05.1992 JP 127672/92
(71) Applicant: TAISHO PHARMACEUTICAL CO. LTD, Tokyo 171 (JP); Senju Pharmaceutical Co., Ltd., Osaka-shi, Osaka 541 (JP)
(72) Inventor: AZUMA, Mitsuyoshi, Nishinomiya-shi, Hyogo 661 (JP); ABE, Yoshimasa, Rm. 310 Adoriimu Uozumi, Akashi-shi, Hyogo 674 (JP); SOGO, Shunji, Yao-shi, Osaka 581 (JP)
(74) Representative: von Kreisler, Alek, Dipl.-Chem.
(86) International application number: JP9300643
(87) International publication number: WO9323032

(57) **Abstract**

The use of (+)-(2S, 3S)-3-[(S)-3-methyl-1-(3-methylbutylcarbamoyl)butylcarbamoyl]-2-oxiranecarboxylic acid represented by formula (I), its salt or its ehtyl ester for treating and preventing cataract, and a process for producing an agent for treating and preventing cataract in the form of eye drops comprising the above compound and an aqueous medium, wherein the pH of the medium is so controlled before or after mixing both the ingredients that the pH of the final preparation will be 3 to 7. This compound is excellent in the effect of treating and preventing cataract. In particular, the above aqueous preparation with the pH of 3 to 7 is an agent for treating and preventing cataract which is excellent in the ability to permeate the cornea and is stable.

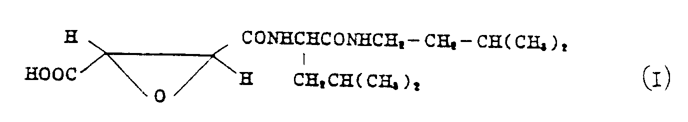

## Description

### Technical Field

The present invention relates to a novel agent comprising (+)-(2S,3S)-3[(S)-3-methyl-1-(3-methylbutylcarbamoyl)butylcarbamoyl]-2-oxiranecarboxylic acid (hereinafter referred to as E-64c), its salt (hereinafter referred to as E-64c salt) or its ethyl ester (hereinafter referred to as E-64d), which is useful for the treatment and prevention of cataract, and to a method for its preparation.

### Background Art

Cataract is a disease where lens becomes opaque due to various factors, which comes out in the form of paropsis as a result of light scatter caused by the opacity of lens, since the scatter leads to an inability to form normal images on retina.

According to the causes, cataract is classified into congenital cataract, senile cataract, complicated cataract, traumatic cataract, diabetic cataract, and so on, and besides these, radiation cataract, glassworker's cataract, etc. are also known, all of which constitute serious eye diseases which accompany lowered eyesight due to the opacity of lens.

Cataract has so far been treated by the administration of various pharmaceutical agents such as antiquinoids (e.g. pirenoxine), agents involved in glutathione synthesis (e.g. cysteine), agents having an SH group (e.g. tiopronin), aldose reductase inhibitors (e.g. tolrestat), or the like. However, administration of these agents is not entirely satisfactory partly due to a markedly slow metabolism of lens. Consequently, the treatment of cataract is currently limited to surgical ones. The surgical treatment is given by translocation of the opacity within the eyeball, or enucleation of the entire lens out from the eyeball. The both methods are burdensome to patients in that incision and suture of cornea, and incisional wound of lens are inevitable even when operated with the use of the state-of-the-art surgery technique.

What is causing cataract is still unknown, but oxidation, decomposition, and agglutination of crystalline protein are deemed to be concerned with the disease, and an increased amount of calcium has been observed in lenses which developed opacity [Invest. Ophthalmol., vol. 13, 204-209 (1974)].

It has been reported that trans-epoxysuccinyl-leucylamide-(4-guanidino)butane (hereinafter referred to as E-64) which is an inhibitor of calpain, a cysteine protease existing in lens which is activated by calcium, can prevent opacity of lens in selenite cataract models and diabetic cataract models of cultured rat lenses [*Atarashii Ganka*, vol. 6, 1573-1576 (1989)]. Yet, E-64 has so weak an effect in cataract treatment that it is not practically used.

It has been also reported that E-64c, an inhibitor of cysteine protease synthesis, its salt and E-64d are effective in the treatment of acute central nervous system injury caused by acute injury and/or circulatory disorders in brain and spinal cord, based on its cysteine protease inhibitory action (Japanese Patent Unexamined Publication No. 255423/1987).

### Disclosure of the Invention

In view of the foregoing situations, the present inventors have conducted intensive studies in search of a superior agent for treating cataract, a preventive thereof, and a method for their preparation, and found that E-64c, E-64c salt and E-64d surprisingly show excellent, unexpected effects in the treatment and prevention of cataract, and established a method for preparing an agent for the treatment and prevention of cataract, which comprises said compound as an active ingredient. The present inventors have also found that E-64c, E-64c salt and E-64d can easily penetrate cornea and exhibit pharmacological activity at a pH in the range of from 3 to 7, and that E-64d is extremely stable at said pH range, which resulted in the completion of the invention.

Accordingly, the present invention relates to:
1. An agent for treating and preventing cataract, which comprises at least one compound selected from the group of E-64c, E-64c salt and E-64d, and preferably has a pH in the range of from 3 to 7 (hereinafter the agent for treating and preventing cataract is referred to as cataract-treating agent);
2. A method for treating and preventing cataract, which comprises administration of an effective amount of at least one compound selected from the group of E-64c, E-64c salt and E-64d (hereinafter the method for treating and preventing cataract is referred to as cataract-treating method);
3. Use of at least one compound selected from the group of E-64c, E-64c salt and E-64d for the preparation of a cataract-treating agent;
4. A method for preparing a cataract-treating agent in the form of eye drops, comprising adjustment of a pH of an aqueous solvent such that the pH of the final preparation falls in the range of from 3 to 7 before or after mixing at least one compound selected from the group of E-64c, E-64c salt and E-64d with the aqueous solvent; and
5. A method for preparing an aqueous liquid containing said compound, comprising adjustment of a pH of an aqueous solvent such that the pH of the final aqueous liquid falls in the range of from 3 to 7 before or after mixing at least one compound selected from the group of E-64c, E-64c salt and E-64d with the aqueous solvent.

The E-64c and E-64d are represented by the following formula 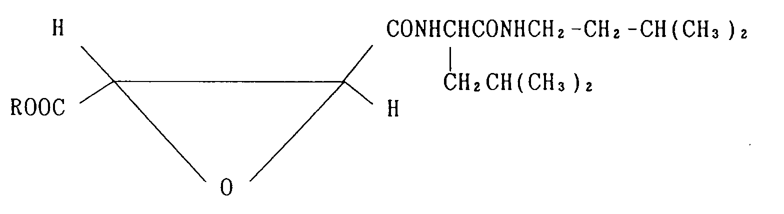
Of the compounds of the formula, a compound wherein R is a hydrogen is E-64c, and a compound wherein R is an ethyl is E-64d.

The salt of E-64c salt is subject to no particular limitation insofar as it is pharmacologically acceptable, and is exemplified by a salt with an alkali metal such as sodium or potassium, a salt with an alkaline earth metal such as calcium or barium, a salt with a basic amino acid such as lysine or arginine, or a salt with an amine such as dimethylamine or triethylamine.

The E-64c, E-64c salt and E-64d can be easily produced by, for example, the method disclosed in Japanese Patent Unexamined Publication No. 115878/1980 (US Patent No. 4382889).

The E-64c, E-64c salt and E-64d to be used in the present invention possess excellent inhibitory effect on opacity of lens, as evidenced by the experiment examples to be mentioned later, and can be used as an efficacious pharmaceutical for the treatment and prevention of cataract.

When the E-64c, E-64c salt or E-64d is used as a cataract-treating agent, they can be formulated into powders, granules, tablets, capsules, injections, eye drops or eye ointments, with preference given to eye drops, eye ointments and injections.

These preparations can be administered orally or parenterally.

The preparation of the present invention can be produced by mixing at least one compound selected from the group of E-64c, E-64c salt and E-64d (hereinafter also simply referred to as compound) and a base.

The base here encompasses pharmacologically acceptable carriers, excipients, diluents, and so on known per se.

Examples of the base for eye drops include aqueous solvents such as sterile purified water, physiological saline, and buffer.

Examples of the base for eye ointments preferably include vaseline, plastibase, liquid paraffin, polyethylene glycol, and carboxymethylcellulose.

In the case of eye drops and eye ointments, E-64d is preferably used as an active ingredient, since this compound can easily penetrate cornea and is quickly hydrolyzed by the enzymes in the eye into E-64c, an active compound thereof, to exhibit desired treatment and preventive effects.

The pH of the eye drops of the invention is normally from 3 to 7, preferably from 4 to 6, more preferably from 4.5 to 5.5. Where the pH is less than 3, side-effects such as disorders in ocular tissue occur and the compound becomes unstable, while where it is more than 7, the compound becomes undesirably unstable.

The E-64c, E-64c salt and E-64d may be dissolved or suspended in a base aqueous solvent.

The additives to be added as appropriate in eye drops are exemplified by the following.

Buffers include, for example, phosphate buffer, borate buffer, citrate buffer, tartrate buffer, acetate buffer, and amino acid. Preferred is a buffer having a buffer capacity in the pH range of 2-9.

Isotonizing agents include, for example, sugars such as sorbitol, glucose and mannitol, polyhydric alcohols such as glycerine, polyethylene glycol and propylene glycol, and salts such as sodium chloride.

Preservatives include, for example, benzalkonium chloride, benzethonium chloride, p-oxybenzoates such as methyl p-oxybenzoate and ethyl p-oxybenzoate, benzyl alcohol, phenethyl alcohol, sorbic acid and its salt, thimerosal, and chlorobutanol.

Thickeners include, for example, hydroxyethylcellulose, hydroxypropylcellulose, methylcellulose, hydroxypropylmethylcellulose, and carboxymethylcellulose and its salt.

Solubilizers (stabilizers) include, for example, water-soluble polymers such as cyclodextrins and polyvinylpyrrolidone, and surfactants such as Polysorbate 80.

Chelating agents include, for example, disodium edetate, sodium citrate, and condensed sodium phosphate.

Suspending agents include, for example, surfactants such as Polysorbate 80, and water-soluble polymers such as sodium methylcellulose, hydroxypropylmethylcellulose and methylcellulose.

Eye drops can be prepared by mixing a base such as an aqueous solvent, additives as described, and at least one compound selected from the group of E-64c, E-64c salt and E-64d in an appropriate order, and adjusting the pH of the mixture to 3-7 by an appropriate step, followed by sterilization by an appropriate step.

The methods for sterilization are conventional and include, for example, sterilization by filtration, high pressure steam sterilization, and flow steam sterilization. Preferred method is sterilization by filtration using a 0.22 µm membrane filter.

An eye drop in which the compound has been dissolved is preferably prepared by adding additives and at least one active ingredient compound selected from the group of E-64c, E-64c salt and E-64d to a base in a suitable order, and adjusting its pH to 3-7. When a buffer is used, the pH is preferably adjusted to 4-6 after the addition of a buffer having a buffer capacity in the pH range of 2-9.

An eye drop in which the compound has been suspended is preferably prepared by adding additives to a base, adjusting its pH to 3-7, subjecting the solution to sterilization, and mixing a compound separately sterilized. When a buffer is used, the pH is preferably adjusted to 4-6 after the addition of a buffer having a buffer capacity in the pH range of 2-9.

The pH adjusting agents to be used here may be conventional ones, and include, for example, hydrochloric acid, acetic acid, phosphoric acid, sodium hydroxide, and ammonium hydroxide, with preference given to 1N hydrochloric acid and 1N sodium hydroxide.

When the cataract-treating agent of the present invention is used in the form of an eye ointment, it is prepared by mixing at least one compound selected from the group of E-64c, E-64c salt and E-64d with a base conventionally employed for eye ointments, and then following the conventional processes.

While the amount of the compound selected from the group of E-64c, E-64c salt and E-64d, which is to be contained in the cataract-treating agent of the present invention varies depending on the kind of the compound to be selected, preparation forms, etc., it is preferably contained in a proportion of about 0.001-10 w/v%, more preferably about 0.01-1 w/v% for eye drops and about 0.001-10 w/w%, more preferably about 0.01-1 w/w% for eye ointments.

When E-64c, E-64c salt or E-64d is used as a cataract-treating agent, the dosage varies depending on the kind of the compound to be used, age of patients, body weight, symptom, preparation form, and so on. When the agent is administered to an adult suffering from cataract, the compound may be contained in an ophthalmic solution or suspension in a proportion of about 0.001-10 w/v%, preferably about 0.01-1 w/v%, and administered 3-5 times a day by one to several drops at a time. In case of an eye ointment, the compound is preferably contained in a proportion of about 0.001-10 w/w%, more preferably about 0.01-1 w/w%, and administered 1-4 times a day according to symptoms.

The cataract-treating agent of the present invention can contain one or more other cataract-treating agents such as antioxidants (e.g. pirenoxine), agents involved in glutathione synthesis (e.g. cysteine), agents having an SH group (e.g. tiopronin), aldose reductase inhibitors (e.g. tolrestat), and the like in suitable combinations to the extent that the object of the invention is not impaired.

The cataract-treating agent of the present invention may contain, along with the various other cataract-treating agents as described, other ingredients having different pharmaceutical effects, insofar as the object of the invention can be achieved.

The cataract-treating agent and the cataract-treating method of the present invention exhibit superior effect in the treatment and prevention of various cataracts.

### Brief Description of the Drawings

Figure 1A and 1B are graphs showing the effect of E-64c by intraperitoneal administration to rats and E-64d by instillation on galactose cataract in rats.

Figure 2 is a graph showing the inhibitory effect of E-64 and E-64d on calcium ionophore-induced cataract in cultured rat lenses relative to the inhibitory effect on the opacity of lenses in the control group.

### Best Mode for Embodying the Invention

The present invention is detailedly described by way of examples and experiment examples in the following, to which the invention is not construed to be limited.

### Example 1

### Ophthalmic suspension

An ophthalmic suspension was prepared according to the following formulation.

| | |
|---|---|
| E-64d | 1 g |
| polyvinyl alcohol | 0.5 g |
| sodium monohydrogenphosphate 12 hydrate | 0.5 g |
| sodium dihydrogenphosphate 2 hydrate | 0.2 g |
| disodium edetate | 0.02 g |
| sodium chloride | 0.7 g |
| benzalkonium chloride | 0.007 g |
| sterile purified water | appropriate amount |
| Total amount | 100 ml |

### (Preparation method)

Polyvinyl alcohol (0.5 g), sodium monohydrogenphosphate 12 hydrate (0.5 g), sodium dihydrogenphosphate 2 hydrate (0.2 g), disodium edetate (0.02 g), sodium chloride (0.7 g) and benzalkonium chloride (0.007 g) were dissolved in 80 ml of sterile purified water, and sterile purified water was further added to make the total amount 100 ml, which was then filtered for sterilization through a membrane filter (0.22 µm) to give a solution (pH 6). E-64d (1 g) previously sterilized was suspended therein to give an ophthalmic suspension.

### Example 2

### Ophthalmic solution

An ophthalmic solution was prepared according to the following formulation.

| | |
|---|---|
| E-64c | 0.1 g |
| boric acid | 1.7 g |
| sodium tetraborate | 0.4 g |
| disodium edetate | 0.02 g |
| benzalkonium chloride | 0.005 g |
| sterile purified water | appropriate amount |
| Total amount | 100 ml |

### (Preparation method)

Boric acid (1.7 g), sodium tetraborate (0.4 g), disodium edetate (0.02 g), benzalkonium chloride (0.005 g) and E-64c (0.1 g) were dissolved in sterile purified water, and sterile purified water was further added to make the total amount 100 ml, which was then filtered for sterilization through a membrane filter (0.22 µm) to give an ophthalmic solution (pH 7).

### Example 3

### Eye ointment

An eye ointment was prepared according to the following formulation.

| | |
|---|---|
| E-64c | 0.5 g |
| liquid paraffin | 1 g |
| white vaseline | appropriate amount |
| Total amount | 100 ml |

### (Preparation method)

Liquid paraffin and white vaseline were previously sterilized by heating. E-64c (0.5 g) was thoroughly triturated with liquid paraffin (1 g), and kneaded with an appropriate amount of white vaseline to give an eye ointment.

### Example 4

### Eye ointment

An eye ointment was prepared according to the following formulation.

| | |
|---|---|
| E-64d | 0.1 g |
| liquid paraffin | 1 g |
| white vaseline | appropriate amount |
| Total amount | 100 ml |

### (Preparation method)

Liquid paraffin and white vaseline were previously sterilized by heating. E-64d (0.1 g) was thoroughly triturated with liquid paraffin (1 g), and kneaded with an appropriate amount of white vaseline to give an eye ointment.

### Example 5

### Ophthalmic solution

An ophthalmic solution was prepared according to the following formulation.

| | |
|---|---|
| E-64c | 1 g |
| sodium acetate | 0.05 g |
| sodium chloride | 0.9 g |
| methyl p-oxybenzoate | 0.026 g |
| propyl p-oxybenzoate | 0.014 g |
| 1N hydrochloric acid | appropriate amount |
| sterile purified water | appropriate amount |
| Total amount | 100 ml |

### (Preparation method)

Sterile purified water (80 ml) was heated to about 80°C, and methyl p-oxybenzoate (0.026 g) and propyl p-oxybenzoate (0.014 g) were dissolved therein. After cooling the solution to room temperature, sodium acetate (0.05 g), sodium chloride (0.9 g) and E-64c (1 g) were dissolved therein, and the solution was adjusted to pH 5 with 1N hydrochloric acid. Sterile purified water was added thereto to make the total amount 100 ml, and the solution was sterilized by filtration through a membrane filter (0.22 µm) to give an ophthalmic solution.

### Example 6

### Ophthalmic solution

An ophthalmic solution was prepared according to the following formulation.

| | |
|---|---|
| E-64c | 1 g |
| sodium dihydrogenphosphate | 0.1 g |
| glycerine | 2.6 g |
| disodium edetate | 0.01 g |
| benzalkonium chloride | 0.005 g |
| 1N hydrochloric acid | appropriate amount |
| sterile purified water | appropriate amount |
| Total amount | 100 ml |

### (Preparation method)

Sodium dihydrogenphosphate (0.1 g) glycerine (2.6 g), disodium edetate (0.01 g), benzalkonium chloride (0.005 g) and E-64c (1 g) were dissolved in sterile purified water (80 ml), and the solution was adjusted to pH 6 with 1N hydrochloric acid. Sterile purified water was added thereto to make the total amount 100 ml, and the solution was sterilized by filtration through a membrane filter (0.22 µm) to give an ophthalmic solution.

### Example 7

### Ophthalmic suspension

An ophthalmic suspension was prepared according to the following formulation.

| | |
|---|---|
| E-64d | 1 g |
| ε-aminocaproic acid | 0.1 g |
| sodium chloride | 0.9 g |
| Polysorbate 80 | 0.1 g |
| benzalkonium chloride | 0.005 g |
| 1N hydrochloric acid | appropriate amount |
| sterile purified water | appropriate amount |
| Total amount | 100 ml |

### (Preparation method)

ε-Aminocaproic acid (0.1 g), sodium chloride (0.9 g), Polysorbate 80 (0.1 g) and benzalkonium chloride (0.005 g) were dissolved in sterile purified water (80 ml), and the solution was adjusted to pH 5 with 1N hydrochloric acid. Sterile purified water was added thereto to make the total amount 100 ml, and the solution was sterilized by filtration through a membrane filter (0.22 µm), after which E-64d (1 g) sterilized beforehand was suspended therein to give an ophthalmic suspension.

### Example 8

### Ophthalmic suspension

An ophthalmic suspension was prepared according to the following formulation.

| | |
|---|---|
| E-64d | 1 g |
| sodium acetate | 0.05 g |
| sodium chloride | 0.9 g |
| methyl p-oxybenzoate | 0.026 g |
| propyl p-oxybenzoate | 0.014 g |
| 1N hydrochloric acid | appropriate amount |
| sterile purified water | appropriate amount |
| Total amount | 100 ml |

### (Preparation method)

Sterile purified water (80 ml) was heated to about 80°C, and methyl p-oxybenzoate (0.026 g) and propyl p-oxybenzoate (0.014 g) were dissolved therein. After cooling the solution to room temperature, sodium acetate (0.05 g) and sodium chloride (0.9 g) were added, and the solution was adjusted to pH 4 with 1N hydrochloric acid. Sterile purified water was added thereto to make the total amount 100 ml, and the solution was sterilized by filtration through a membrane filter (0.22 µm). Then, E-64d (1 g) was suspended therein to give an ophthalmic suspension.

### Example 9

### Eye ointment

An eye ointment was prepared according to the following formulation.

| | |
|---|---|
| E-64d | 0.1 g |
| liquid paraffin | 2 g |
| white vaseline | appropriate amount |
| Total amount | 100 ml |

### (Preparation method)

Liquid paraffin and white vaseline were previously sterilized by heating. E-64d (0.1 g) was thoroughly triturated with liquid paraffin (2 g), and kneaded with an appropriate amount of white vaseline to give an eye ointment.

### Example 10

### Eye ointment

An eye ointment was prepared according to the following formulation.

| | |
|---|---|
| E-64c | 0.1 g |
| liquid paraffin | 2 g |
| plastibase | appropriate amount |
| Total amount | 100 ml |

### (Preparation method)

Liquid paraffin and plastibase were previously sterilized by heating. E-64c (0.1 g) was thoroughly triturated with liquid paraffin (2 g), and kneaded with an appropriate amount of plastibase to give an eye ointment.

### Experimental Example

The pharmacological tests and results showing effectiveness of the cataract-treating agent of the present invention are given in the following.

### Experiment Example 1

### Effect of intraperitoneal administration of E-64c and instillation of E-64d on galactose cataract in rats

Three-week-old male SD rats (n=30) were bred in a breeding room having a temperature of 24±4°C and humidity of 55±15%, on Labo MR stock (manufactured by Nihon Nosan Kogyo Co., Ltd., Japan) and tap water.

As the test drugs, used were (1) 1% solution of E-64c prepared by dissolving E-64c bulk powder in 0.1N sodium hydroxide, adjusting the pH to 5 with 0.1N hydrochloric acid, and adding purified water; and (2) 1% suspension of E-64d prepared by suspending E-64d bulk powder in 0.3% Tween 80, the osmotic pressure of both of which having been adjusted to 290 mOsm/kg H₂O with sodium chloride. As a control, used was (3) physiological saline.

The test animals were pre-bred for a week, and then bred on a 25% galactose feed (solidified product of a mixture of galactose and Labo MR stock powder at the ratio of 1:3). The animals were fed for 24 hours daily for the first 7 days, and 9 hours a day from the 8th day. The test drugs were administered from the day when galactose feeding was initiated, and (1) was administered intraperitoneally once a day (100 mg/kg, 1 ml/100g as liquid), and (2) was instilled only in the right eye 4 times a day by 5 µl at a time without treatment to the left eye. The control group was instilled with (3) in the same manner as in the case of (2) without treatment to the left eye.

Lenses were observed under mydriasis using a slit lamp (SL-5D) at 7 day-intervals from the initiation of the test drug and physiological saline (control) administrations until day 28, and the progression of cataract up to the mature cataract was evaluated according to the following 8 stages of cataract grades defined by Sippel [Invest. Ophthalmol., vol. 6, 568-575 (1966)].

| grade | progression of cataract |
|---|---|
| 0 | Clear |
| 0.5 | Progressive vacuolization of an equatorial band |
| 1 | Progressive vacuolization of 1/3 of anterior cortex |
| 1.5 | Progressive vacuolization of 2/3 of anterior cortex |
| 2 | Continued enlargement and coalescence of vacuole |
| 3 | Uniform opalescence or posterior opacification |
| 4 | Nuclear cataract |
| 5 | Total opacification |

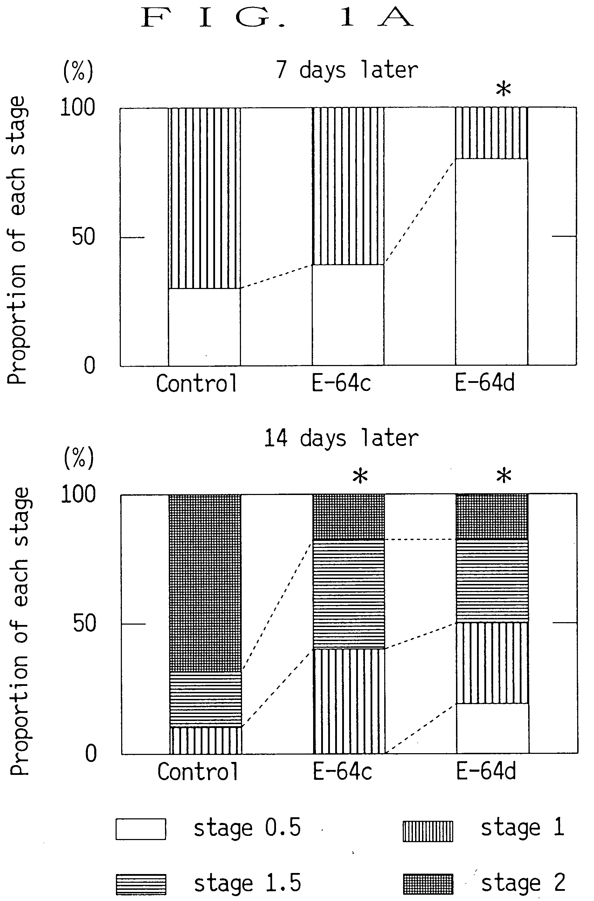

The proportion of each stage at the time of cataract observation of the test animals administered with physiological saline or respective test drugs (E-64c or E-64d) is shown in Fig. 1A,B. In Fig. 1A,B, the axis of ordinate shows the proportion of each stage according to the grades of Sippel at respective observation periods, wherein significant difference from the control group is expressed as : + ; 0.05<P<0.10, * ; P<0.01.

As is evident from the results in Fig. 1A,B, determination according to Wilcoxon method revealed that significant delay of cataract was observed at 14 days from the initiation of the administration in the group administered with E-64c; and in the group administered with E-64d, significant delay of cataract was observed at 7 and 14 days from the initiation of the administration and delaying tendency at 21 days from the initiation of the administration. The time series variance analysis revealed that the group administered with E-64c or E-64d showed significant delay of cataract as compared with the control group.

### Experiment Example 2

### Effect of E-64d on calcium ionophore-induced cataract in cultured rat lenses

Lenses were dissected from 4-week-old male S.D. rats, and cultured for one day in (1) a basic culture medium (MEM), (2) a culture medium of (1) supplemented with 10 µM calcium ionophore (A23187), or (3) a culture medium of (2) supplemented with 4, 20, 100 or 500 µM E-64 or E-64d. Thereafter, the medium was changed as follows: (1) basic medium, (2) basic medium, and (3) basic medium with several concentrations of E-64 or E-64d, and culture was continued for 4 more days, after which opacity of lenses was measured using an image analyser.

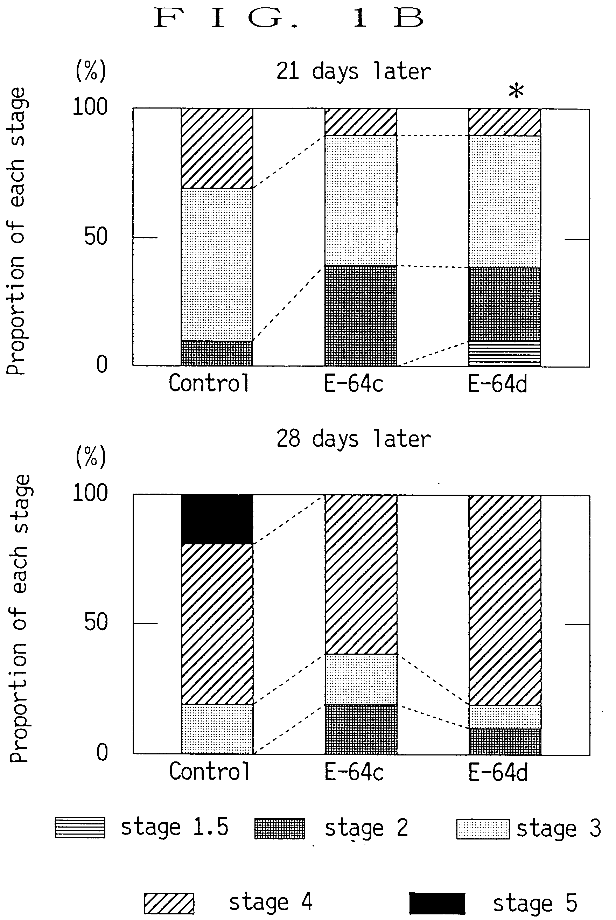

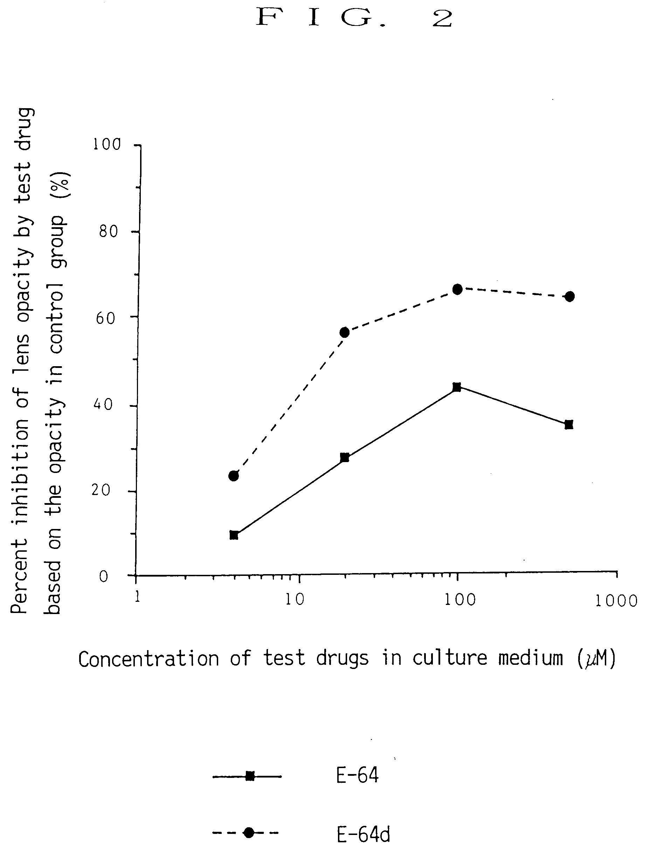

The ratio of the inhibition of the development of lens opacity by each test drug (E-64, E-64d) to that in the control group is shown in Fig. 2. In Fig. 2, the axis of abscissa shows the concentration of each drug in the culture medium (µM), and the axis of ordinate shows percent inhibition of lens opacity by each test drug based on the opacity in the control group.

As is evident from the results of Fig. 2, E-64 or E-64d inhibited the opacity of lens caused by calcium ionophore in a concentration-dependent manner. The inhibition effect was stronger in E-64d than in E-64, and the opacity of lens was inhibited by 50% or more at a concentration of 20 µM.

### Experiment Example 3

### Effect of intraperitoneal administration of E-64d on cataract in Nakano mouse

Thirty-five Nakano mice (9 days old) were divided into three groups, and each group was intraperitoneally administered once a day with E-64d at 30 mg/kg or 100 mg/kg, or 5% gum arabic solution as a control. E-64d was suspended in 5% gum arabic solution at a concentration of 0.15% or 0.5%, respectively, and administered to the mice. After the eyes of the mice opened, opacity of the lens was observed using a slip lamp to evaluate progress of cataract.

As a result, the control group showed opacity in the nuclear lens when the mice were 21 days old, and the opacity was found in about 40% of the mice. In contrast, about 12% of the group administered with E-64d (30 mg/kg) showed nuclear opacity, and no development of opacity was observed in the group administered with E-64d (100 mg/kg). The time-course observation showed that the nuclear opacity in the group administered with E-64d was limited to a smaller degree as compared with the control group.

### Experiment Example 4

### Effects of pH on corneal permeability of E-64c (in vitro)

Corneal permeability was tested *in vitr*o using a side-by-side type permeation cell equipped with cornea from albino rabbit. A 1% E-64c solution prepared using a buffer having the following formulation was charged in a donor cell, and the following buffer was charged in a receptor cell, and they were incubated at 34°C. The amount of E-64c permeated into the receptor cell was measured with time. The accumulative amount of the permeated E-64c was measured 1, 2, and 3 hours later. As a result, E-64c permeated through the cornea at a pH not more than 7, and its permeability was pH-dependent. In the pH range of 3 or below, tissue disorders causing corneal opacity were observed.

| Buffer formulation | |
|---|---|
| calcium chloride | 0.175 g |
| potassium chloride | 0.4 g |
| magnesium sulfate | 0.4 g |
| phosphoric acid sodium dihydride | 0.187 g |
| sodium chloride | 7.97 g |
| glucose | 1 g |
| purified water | 1000 ml |

**Table 1**

| corneal permeability of E-64c *in vitro* | | | |
|---|---|---|---|
| | permeability (µg/cm²) | | |
| | 1 h | 2 h | 3 h |
| pH 7 | 1.69 | 6.15 | 13.78 |
| pH 5 | 63.9 | 309.6 | 611.6 |
| pH 3 | 145.6 | 790.4 | 1479.9 |

### Experiment Example 5

### Effects of pH on corneal permeability of E-64d (in vitro)

In the same manner as in Experiment Example 4, corneal permeability of a 1% E-64d suspension (pH 7) was examined. E-64d was not detected in a receptor solution, but E-64c produced by hydrolysis by the enzymes in cornea was detected from immediately after the initiation of the experiment. The amount of the metabolized product E-64c detected in the receptor cell 20 minutes, 1 h, 2 h or 3 h later was 2.8, 5.8, 10.3, and 21.4 µg/cm², respectively.

### Experiment Example 6

### pH stability of E-64d

The stability of 1% E-64d suspensions adjusted to various pHs was examined. The results of the stability test is shown in Table 2. It was found that E-64d was stable in the vicinity of pH 4-6.

**Table 2**

| Stability of E-64d | | |
|---|---|---|
| | residual rate (%) | |
| | 40°C (4 wk) | 60°C (1 wk) |
| pH 4 | 96.6 | 98.7 |
| pH 5 | 99.8 | 98.7 |
| pH 6 | 95.2 | 97.9 |
| pH 7 | 75.7 | |

## Claims

1. An agent for treating and preventing cataract, which comprises at least one compound selected from the group consisting of (+)-(2S,3S)-3[(S)-3-methyl-1-(3-methylbutylcarbamoyl)butylcarbamoyl]-2-oxiranecarboxylic acid, its salt, and its ethyl ester.

2. The agent of Claim 1, wherein the compound is (+)-(2S,3S)-3[(S)-3-methyl-1-(3-methylbutylcarbamoyl)butylcarbamoyl]-2-oxiranecarboxylic acid or its salt.

3. The agent of Claim 1, wherein the compound is an ethyl ester of (+)-(2S,3S)-3[(S)-3-methyl-1-(3-methylbutylcarbamoyl)butylcarbamoyl]-2-oxiranecarboxylic acid.

4. The agent of Claim 1, which is in the form of an eye drop.

5. The agent of Claim 4, which is an eye drop comprising the compound in a proportion of 0.001-10 w/v%.

6. The agent of Claim 4 or Claim 5, which has a pH of from 3 through 7.

7. The agent of Claim 1, which is in the form of an eye ointment.

8. The agent of Claim 7, which is an eye ointment comprising the compound in a proportion of 0.001-10 w/w%.

9. A method for treating and preventing cataract, comprising administering a therapeutically effective amount of at least one compound selected from the group consisting of (+)-(2S,3S)-3[(S)-3-methyl-1-(3-methylbutylcarbamoyl)butylcarbamoyl]-2-oxiranecarboxylic acid, its salt, and its ethyl ester.

10. The method of Claim 9, comprising administering the compound in the form of an eye drop.

11. The method of Claim 10, wherein the compound is comprised in the eye drop in a proportion of 0.001-10 w/v%.

12. The method of Claim 10, wherein the pH of the eye drop is from 3 through 7.

13. The method of Claim 9, comprising administering the compound in the form of an eye ointment.

14. The method of Claim 13, wherein the compound is comprised in the eye ointment in a proportion of 0.001-10 w/w%.

15. Use of one compound selected from the group consisting of (+)-(2S,3S)-3[(S)-3-methyl-1-(3-methylbutylcarbamoyl)butylcarbamoyl]-2-oxiranecarboxylic acid, its salt, and its ethyl ester for the preparation of an agent for the treatment and prevention of cataract.

16. The use of Claim 15, wherein the agent for the treatment and prevention of cataract is in the form of an eye drop.

17. The use of Claim 16, wherein the compound is comprised in the eye drop in a proportion of 0.001-10 w/v%.

18. The use of Claim 16, wherein the pH of the eye drop is from 3 through 7.

19. The use of Claim 15, wherein the agent for the treatment and prevention of cataract is in the form of an eye ointment.

20. The use of Claim 19, wherein the compound is comprised in the eye ointment in a proportion of 0.001-10 w/w%.

21. A method for preparing an agent for treating and preventing cataract, comprising mixing at least one compound selected from the group consisting of (+)-(2S,3S)-3[(S)-3-methyl-1-(3-methylbutylcarbamoyl)butylcarbamoyl]-2-oxiranecarboxylic acid, its salt and its ethyl ester, with a base.

22. The method of Claim 21, wherein the agent for treating and preventing cataract is in the form of an eye drop.

23. The method of Claim 21, comprising adjusting a pH of an aqueous solvent such that the pH of the final preparation falls in the range of from 3 through 7, before or after mixing the compound with the aqueous solvent.

24. The method of Claim 22, comprising adjusting the pH of the aqueous solvent such that the pH of the final preparation falls in the range of from 4 through 6, before or after mixing the compound with the aqueous solvent.

25. The method of Claim 24, comprising adjusting the pH of the final preparation to 4-6 after the addition of a buffer having a buffer capacity in the pH range of 2-9 by an optional step.

26. The method of any one of Claim 23 to Claim 25, further comprising a process of sterilization.

27. The method for preparing an aqueous solution comprising at least one compound selected from the group consisting of (+)-(2S,3S)-3[(S)-3-methyl-1-(3-methylbutylcarbamoyl)butylcarbamoyl]-2-oxiranecarboxylic acid, its salt and its ethyl ester, comprising adjusing a pH of an aqueous solvent such that the pH of the final aqueous preparation falls within the range of from 3 through 7, before or after mixing the compound with the aqueous solvent.

28. The method of Claim 27, comprising adjusting the pH of the aqueous solvent such that the pH of the final aqueous preparation falls within the range of from 4 through 6, before or after mixing the compound with the aqueous solvent.

29. The method of Claim 28, comprising adjusting the pH to 4-6 after the addition of a buffer having a buffer capacity in the pH range of 2-9 by an optional step.

30. The method of any one of Claim 27 to Claim 29, further comprising a process of sterilization.
